# EUROPEAN PATENT SPECIFICATION

(11) **EP 2 044 029 B1**
(45) Date of publication and mention of the grant of the patent: **26.01.2011**
(21) Application number: 07735013.0
(22) Date of filing: 09.07.2007
(51) Int. Cl.: C07D 221/04, A61K 31/496, A61P 25/00

(54) **TARTRATE SALT OF (7S)-7-[(5-FLUORO-2-METHYL-BENZYL)OXY]-2-[(2R)-2-METHYLPIPERAZIN-1-YL]-6,7-DIHYDRO-5H-CYCLOPENTA[B]PYRIDINE**
TARTRATSALZ VON (7S)-7-[(5-FLUOR-2-METHYLBENZYL)OXY]-2-[(2R)-2-METHYLPIPERAZIN-1-YL]-6,7-DIHYDRO-5H-CYCLOPENTA[B]PYRIDIN
SEL DE TARTRATE DE (7S)-7-[(5-FLUORO-2-MÉTHYL-BENZYL)OXY]-2-[(2R)-2-MÉTHYLPIPÉRAZIN-1-YL]-6,7-DIHYDRO-5H-CYCLOPENTA[B]PYRIDINE

(30) Priority: 14.07.2006 US 830890 P
(43) Date of publication of application: 08.04.2009
(73) Proprietor: Pfizer Products Inc., Groton, CT 06340 (US)
(72) Inventor: LIU, Kevin Kun-Chin, San Marcos, CA 92078 (US); ZENG, Yuan, Waterford, CT 06385 (US)
(74) Representative: Hodge, Emma Jane
(86) International application number: PCT/IB2007/002025
(87) International publication number: WO 2008/010073

(56) References cited:
- WO-A-00/12475
- WO-A-2006/103511
- BISHOP M J ET AL: "NEW 5-HT2C RECEPTOR AGONISTS" EXPERT OPINION ON THERAPEUTIC PATENTS, ASHLEY PUBLICATIONS, GB, vol. 13, no. 11, 2003, pages 1691-1705, XP001187592 ISSN: 1354-3776

## Description

### Field of the Invention

The present invention relates to a tartrate salt of (7S)-7-[(5-fluoro-2-methylbenzyl)oxy]-2-[(2R)-2-methylpiperazin,-1-yl]-6,7-dihydro-5H-cyclopenta[b]pyridine, which is a 5HT_{2c} receptor agonist. The present invention also relates to its use in the treatment of diseases linked to activation of the 5HT_{2c} receptor in animals including humans.

### Background of the Invention

Bishop, M. J. and Nilsson, B. M., "New 5-HT2c Receptor Agonists" Expert Opin. Ther. Patents, 2003, 13(11): 1691-1705, review patent applications that describe compounds having agonist activity at the 5-HT_{2c}, receptor. The review also addresses indications for which evidence exists to support the use of 5-HT_{2c} agonists in their treatment, such as obesity, schizophrenia, anxiety, depression, obsessive-compulsive disorder, sexual dysfunction, epilepsy, and urinary incontinence, among others.

Toxicity and non-selectivity of ligands for the various 5-HT receptors remain a challenge. It is suspected that the non-selectivity of some ligands contributes to various adverse side effects such as hallucinations and cardiovascular complications. Therefore, there remains a need for 5-HT_{2c} selective receptor ligands.

### Brief Description of the Drawings

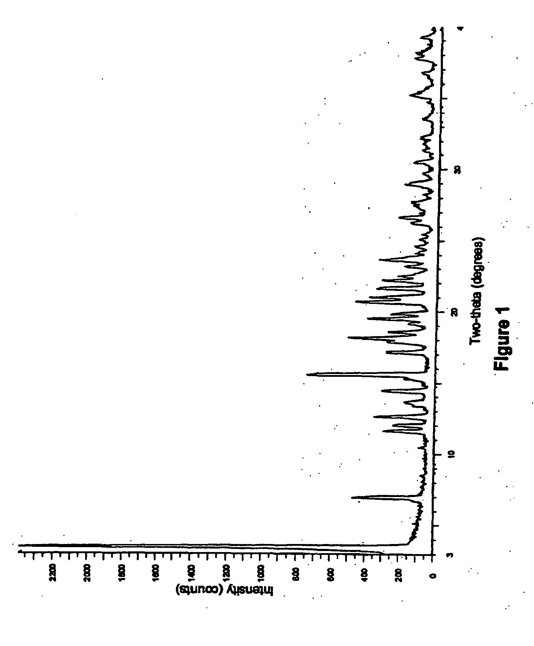

FIG. 1 is the X-ray powder diffraction pattern of (7S)-7-[(5-fluoro-2-methylbenzyl)oxy]-2-[(2R)-2-methylpiperazin-1-y)]-6,-dihydro-5H-cyclopenta[b]pyridine tartrate Form A.

### Summary of the Invention

The present invention provides a tartrate salt of Formula **I:** 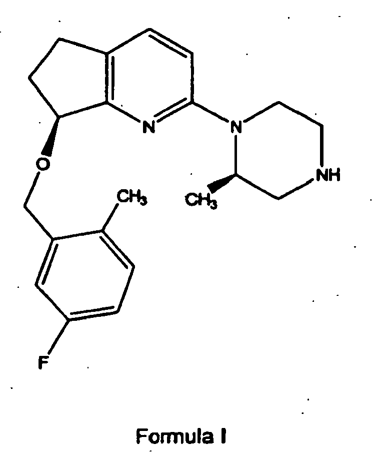 wherein the salt has characteristic x-ray powder diffraction peaks as measured with Cu radiaton of 2-Theta ± 0.2° of 3.5°, 7.0°, 15.6°, 18.1° and 20.7°.

Formula I is known as (7S)-7-[(5-fluoro-2-methyl-benzyt)oxy]-2-[(2R)-2-methylpiperazin -1-yl]-6,7-dihydro-5H-cyclopenta[b]pyridirte. The tartrate salt of Formula I of the present invention is crystalline and relatively non-hygroscopic. The crystalline tartrate salt of Formula I has been found to exist in polymorphic Form A.

The tartrate salt of Formula I of the invention is useful in the treatment of diseases linked to activation of the 5HT_{2c} receptor in animals including humans, for example, in the treatment of schizophrenia, cognitive deficits including cognitive deficits associated with schizophrenia, anxiety, depression, obsessive-compulsive disorder, epilepsy, obesity, sexual dysfunction, and urinary incontinence, among others.

### Detailed Description of the Invention

5HT_{2c} agonists are described in US Application Ser. No. 11/395327 filed March 31, 2006, which is incorporated by reference herein in its entirety.

The term "treatment", as used herein, refers to reversing, alleviating, inhibiting the progress of, or preventing the disorder or condition to which such term applies, or one or more symptoms of such condition or disorder. The term "treatment", as used herein, refers to the act of treating, as "treating" is defined immediately above.

The tartrate salt of Formula I may exist in different polymorphic forms.

The tartrate salt of Formula I has been found to exist in crystalline polymorphic Form A.

The tartrate salt of Formula I of the present invention encompasses solvates or hydrates thereof. The salt may form solvates or hydrates with solvents such as, but not limited to, water, acetone, and alcohol such as ethanol, propanol, butanol, propylene glycol, etc.

The tartrate salt has characteristic x-ray powder diffraction peaks as measured with copper radiation of 2-Theta ± 0.2° of 3.5°, 7.0°, 15.6 °, 18.1°, and 20.7°_{.}

In a preferred embodiment, the tartrate salt has the characteristic X-ray powder diffraction pattern of FIG. 1.

In a further embodiment, the tartrate salt has a melting onset temperature of 186 ± 3°C.

In another embodiment, the tartrate salt increases in weight by less than 0.5% at 90 ± 2% relative humidity in an isothermal (24.9 ± 0.1 °C) moisture sorption test conducted from approximately 1% to 90% (±2%) humidity.

Hygroscopic drug substances become moist due to their affinity for moisture in the air. Highly hygroscopic or deliquescent compounds cannot be prepared satisfactorily as powders. Nonhygroscopic drug substances are preferable for preparing solid unit dosage forms.

As used herein, "crystalline" means a material that has an ordered, long range molecular structure. The degree of crystallinity of a crystal form can be determined by many techniques including, for example, powder X-ray diffraction, moisture sorption, differential scanning calorimetry, solution calorimetry, and dissolution properties.

Crystalline organic compounds consist of a large number of atoms that are arranged in a periodic array in three-dimensional space. The structural periodicity normally manifests distinct physical properties, such as sharp, explicit spectral features by most spectroscopic probes (e.g., X-ray diffraction, infrared and solid state NMR). X-ray diffraction (XRD) is acknowledged to be one of the most sensitive methods to determine the crystallinity of solids. Crystals yield explicit diffraction maxima that arise at specific angles consistent with the lattice interplanar spacings, as predicted by Bragg's law. On the contrary, amorphous materials do not possess long-range order. They often retain additional volume between molecules, as in the liquid state. Amorphous solids normally unveil a featureless XRD pattern with broad, diffuse halos because of the absence of the long range order of repeating crystal lattice.

PXRD has reportedly been used to characterize different crystal forms of organic compounds (e.g., compounds useful in pharmaceutical compositions). See, for example, U.S. Pat. Nos. 5,504,216 (Holohan et al), 5,721,359 (Dunn et al.), 5,910,588 (Wangnick et al.). 6.066.647 (Douglas et al.), 6,225,474 (Matsumoto et al.), 6,239,141 (Allen et al.), 6,251,355 (Murata et al.), 6,288,057 (Harkness), 6,316,672 (Stowell et al.), and 6,329,364 (Groleau).

Crystalline materials are preferred in many pharmaceutical applications. Crystalline forms are generally thermodynamically more stable than amorphous forms of the same substance. This thermodynamic stability is preferably reflected in the lower solubility and improved physical stability of the crystalline form. The regular packing of the molecules in the crystalline solid preferably denies the incorporation of chemical impurities. Hence crystalline materials generally possess higher chemical purity than their amorphous counterparts. The packing in the crystalline solid generally constrains the molecules to well defined lattice positions and reduces the molecular mobility that is the prerequisite for chemical reactions. Hence, crystalline solids, with very few notable exceptions, are chemically more stable than amorphous solids of the same molecular composition.

The crystalline form of the crystalline polymorph of the tartrate salt Form A of Formula I of the present invention has the distinct powder X-ray diffraction profile provided in FIG 1. Characteristic diffraction peaks as used herein are peaks selected from the most intense peaks of the observed diffraction pattern. Preferably, the characteristic peaks are selected from about 20 of the most intense peaks, more preferably from about 10 of the most intense peaks, and most preferably from about 5 of the most intense peaks in the diffraction pattern.

### Powder X-Ray Diffraction Pattern

Powder x-ray diffraction pattern was collected for the tartrate salt of (7S)-7-[(5-fluoro-2-methyl-benzyl)oxy]-2-[(2R)-2-methylpiperazin-1-yl]-6,7-dihydro-5H-cyclopenta[b]pyridine Form A using a Bruker D5000 diffractometer (Madison Wisconsin) equipped with a copper radiation source, fixed slits (divergence 1.0 mm, antiscatter 1.0 mm, and receiving 0.6 mm) and a Kevex solid-state detector. Data was collected in the theta-two theta goniometer configuration from a flat plate sample holder at the Copper wavelength Kα₁ =1.54056 and Kα₂ = 1.54439 from 3.0 to 40.0 degrees two-theta using a step size of 0.040 degrees and a step time of one second. X-ray tube voltage and amperage were set at 40kV and 40mA respectively. Data were collected and analyzed using Bruker DIFFRAC Plus software. Samples were prepared by placing them in a quartz holder. (It is noted that a Bruker D5000 diffractometer is similar in operation to Siemans model D5000.) The results are summarized in Table 1 which provides the two-theta values and relative intensities for all of the reflections (lines) that have a relative intensity greater than or equal to 9% using a smoothing width of 0.30 and a threshold of 1.0.

**Table 1:**

| **Powder X-Ray Diffraction Reflections for the Tartrate Salt Form A of Formula I** | |
|---|---|
| | Relative |
| Angle | Intensity* |
| 2-Theta ± 0.2° | % |
| 3.5 | 100 |
| 7.0 | 19.6 |
| 11.6 | 12.3 |
| 12.0 | 9.8 |
| 12.6 | 14.5 |
| 14.5 | 12.6 |
| 15.6 | 31.0 |
| 17.2 | 11.7 |
| 17.9 | 11.7 |
| 18.1 | 21.2 |
| 18.6 | 11.3 |
| 19.5 | 16.7 |
| 19.8 | 10.9 |
| 20.7 | 19.6 |
| 21.1 | 16.3 |
| 21.7 | 14.6 |
| 22.2 | 13.1 |
| 23.7 | 14.0 |
| 26.7 | 9.3 |

| | |
|---|---|
| * The relative intensity may vary depending on particle size and shape. | |

### Differential Scanning Calorimetry (DSC)

Thermal phase transition data was collected using a Mettler Toledo DSC 822. Crimped Aluminum sample pans with a pinhole in the lid were loaded with one to three milligrams of sample and then scanned from room temperature to 300°C at 5°C/minute. Onset temperatures were determined by baseline tangent peak tangent method. Onset temperatures may vary depending on particle size; sample size, sample pan configuration, and heating rate.

The thermal analysis (DSC and hot stage polarized light microscopy) for the tartrate salt of (7S)-7-[(5-fluoro-2-methyl-benzyl)oxy]-2-[(2R)-2-methylpiperazin-1-yl]-6,7-dihydro-5H-cyclopenta[b]pyridine Form A indicates that it is high melting.. Melting onset = 186 ± 3°C.

### Hygroscopicity

Hygroscopicity was assessed using a dynamic vapor sorption technique in which an accurately weighed sample is subjected to progressively changing water vapor pressure while simultaneously recording the weight change. The experiment was conducted isothermally at 25°C.

Less than 0.5% increase in weight was detected at 90 ± 2% relative humidity during an isothermal (25.1 ± 0.1 °C) moisture sorption analysis conducted from approximately 1% to 90% (±2%) humidity. The dynamic hygroscopicity data generated suggests that the tartrate salt of (7S)-7-[(5-fluoro-2-methyl-benzyl)oxy]-2-[(2R)-2-methylpipeirazin-1-yl]-6,7-dihydro-5H-cyclopenta[b]pyridine Form A is non-hygroscopic. The amount of water vapor sorption may vary depending on the particle size and surface area of sample.

### Example 1

### (S)-7-(5-Fluoro-2-methyl-benzyloxy)-2-((R)-2-methyl-piperazin-1-yl)-6,7-dihydro-5H-[1]pyrindine L(+)-tartrate

L-tartaric acid 41.8 mg (0.279 mmol) was dissolved in 2 mL hot isopropyl alcohol and then cooled to 25 °C. A solution of (7S)-7-[(5-fluoro-2-methyl-benzyl)oxy]-2-[(2R)-2-methylpiperazin-1-yl]-6,7-dihydro-5H-cyclopenta[b]pyridine (Formula I) in accordance with Example 2A-9 of US Serial No. 11/395327 filed March 31, 2006, which is incorporated by reference herein in its entirety (100 mg, 0.281 mmol) in 3 mL isopropyl alcohol was added to the L-tartaric acid solution dropwise via a syringe at 25 °C. Another 1 mL isopropyl alcohol was used to rinse the vial containing Formula I and then added to the L-tartaric acid solution. The resulting mixture was allowed to stir vigorously for 2 hours, and then filtered and washed with ice cold isopropyl alcohol. The white solid collected (113.3 mg) was then mixed with 2.5 mL absolute ethanol and stirred over 72 hours at 80°C. The resulting precipitate was cooled to room temperature, filtered and washed with ice cold absolute ethanol to give 90 mg of title compound as white crystals after drying under vacuum.

### General Preparation of Salts of Formula I

A solution of counter ion (acid) at from about 20°C to 50°C was dissolved in a suitable solvent and added to a solution of the free base of Formula I at from about 20°C to about 50°C in a suitable solvent. The mixture was stirred while cooling to room temperature followed by filtration. Various salts of Formula I were prepared.

The L-(+)-tartrate salt of Formula I was observed to be fully crystalline.

The tartrate salt of Formula I of the present invention is a selective 5-HT_{2c} agonist. The salt may be used to treat diseases or conditions that are effectively treated by agonism of the 5-HT_{2c} receptor. The salt may be used to treat 5-HT₂ receptor-mediated diseases.

An embodiment of the present invention is a pharmaceutical composition comprising a therapeutically effective amount of a tartrate salt of the present invention and a pharmaceutically acceptable carrier and optionally, a pharmaceutically acceptable excipient or diluent. Preferably, the tartrate salt of the pharmaceutical composition is crystalline. The pharmaceutical compositions may be used to treat 5-HT₂ receptor-mediated diseases.

A typical formulation is prepared by mixing a tartrate salt of the present invention and a carrier, and optionally, a diluent or excipient. Suitable carriers, diluents and excipients are well known to those skilled in the art and include materials such as carbohydrates, waxes, water soluble and/or swellable polymers, hydrophilic or hydrophobic materials, gelatin, oils, solvents, water, and the like. The particular carrier, diluent or excipient used will depend upon the means and purpose for which the compound of the present invention is being applied. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG400, PEG300), etc. and mixtures thereof. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug (i.e., a tartrate salt of the present invention or pharmaceutical composition thereof) or aid in the manufacturing of the pharmaceutical product (i.e., medicament).

The formulations may be prepared using conventional dissolution and mixing procedures. For example, the bulk drug substance (i.e., tartrate salt of the present invention or stabilized form (e.g., complex with a cyclodextrin derivative or other known complexation agent)) is dissolved in a suitable solvent in the presence of one or more of the excipients described above. The tartrate salt of the present invention is typically formulated into pharmaceutical dosage forms to provide an easily controllable dosage of the drug and to give the patient an elegant and easily handleable product.

A pharmaceutical composiiton of the present invention can be administered to a patient in any conventional oral, rectal, transdermal, parenteral, (for example, intravenous, intramuscular, or subcutaneous) intracisternal, intravaginal, intraperitoneal, intravesical, local (for example, powder, ointment or drop), or buccal, or nasal, dosage form.

The present invention further provides methods of treating 5-HT₂ receptor-mediated diseases, conditions, or disorders in an animal in need of such treatment that include administering to the animal (preferably, a human) a therapeutically effective amount of a tartrate salt of the present invention or a pharmaceutical composition comprising an effective amount of a tartrate salt of the present invention and a pharmaceutically acceptable carrier.

Formula I of the present invention acts as patent, high affinity agonist at the 5-HT_{2c} receptor, as an antagonist or weak partial agonist at the 5-HT₂ₐ receptor, and as an antagonist at the 5-HT_{2b} receptor. Formula I of the present invention is functionally selective for 5-HT_{2c} against 5-HT₂ₐ and 5-HT_{2b}, by virtue of its much greater agonistic potency (lower EC₅₀) for 5-HT_{2c} than that observed for 5-HT₂ₐ and/or 5-HT_{2b} or its lack of agonistic activity at 5-HT₂ₐ and/or 5-HT_{2b}.

An amorphous salt of Formula I was tested in accordance with the binding assay procedures at human receptors provided in US Application Ser. No. 11/395327, and the following receptor binding data for the compound of Formula I was obtained:
K1 for 5HT_{2c} - 1.5nM;
K₁ for 5HT₂ₐ - 4.5nM;
K₁ for 5HT_{2b} - 7.7nM.

Receptor binding data or binding selectivity data may not always correlate with or reflect functional data or functional selectivity data. For example, a compound may be selective for the 5-HT_{2c} receptor when functional assays are analyzed, but in the binding assays the compound may have the same potency at other 5-HT receptors. Thus, the term "selective" as used herein in relation to the present invention with respect to methods of treatment means "functionally selective".

Accordingly, the tartrate salt of Formula I of the present invention is useful in treating 5-HT₂ receptor-mediated diseases, conditions, or disorders. Consequently, the tartrate salt of the present invention may be used in the manufacture of a medicament for the therapeutic applications described herein.

Diseases, conditions, and/or disorders modulated by 5HT₂ receptor ligands include eating disorders (e.g., binge eating disorder, anorexia, and bulimia), weight loss or control (e.g., reduction in calorie or food intake, and/or appetite suppression), obesity, depression, atypical depression, bipolar disorders, psychoses, schizophrenia, behavioral addictions, suppression of reward-related behaviors (e.g., conditioned place avoidance, such as suppression of cocaine- and morphine-induced conditioned place preference), substance abuse, addictive disorders, impulsivity, alcoholism (e.g., alcohol abuse, addiction and/or dependence including treatment for abstinence, craving reduction and relapse prevention of alcohol intake), tobacco abuse (e.g., smoking - addiction, cessation and/or dependence including treatment for craving reduction and relapse prevention of tobacco smoking), premenstrual syndrome or late luteal phase syndrome, migraine, panic disorder, anxiety, post-traumatic syndrome, dementia (including memory loss, Alzheimer's disease, dementia of aging, vascular dementia, mild cognitive impairment, age-related cognitive decline, and mild neurocognitive disorder), seizure disorders, epilepsy, gastrointestinal disorders (e.g., dysfunction of gastrointestinal motility or intestinal propulsion), attention deficit disorders or attention hyperactivity disorders (ADD/ADHD), disruptive behavior disorders, impulse control disorders, borderline personality disorder, obsessive compulsive disorder, chronic fatigue syndrome, anorexia nervosa, disorders of sleep (e.g., sleep apnea), autism, epilepsy, mutism, spinal cord injury, damage of the central nervous system (e.g., trauma, stroke, neurodegenerative diseases or toxic or infective CNS diseases (e.g., encephalitis or meningitis)), cardiovascular disorders (e.g., thrombosis), Parkinson's disease, diabetes insipidus, and type II diabetes.

In another embodiment, this invention relates to a method for treating psychotic disorders and conditions such as schizophrenia, delusional disorders and drug induced psychosis; anxiety disorders such as panic and obsessive-compulsive disorder; and movement disorders including Parkinson's disease and Huntington's disease, comprising an amount of a tartrate salt of Formula I effective in treating said disorder or condition.

Examples of psychotic disorders that can be treated according to the present invention include, but are not limited to, schizophrenia, for example of the paranoid, disorganized, catatonic, undifferentiated, or residual type; schizophreniform disorder; schizoaffective disorder, for example of the delusional type or the depressive type; delusional disorder; substance-induced psychotic disorder, for example psychosis induced by alcohol, amphetamine, cannabis, cocaine, hallucinogens, inhalants, opioids, or phencyclidine; personality disorder of the paranoid type; and personality disorder of the schizoid type.

In use to treat psychotic disorders of the schizophrenic types, the tartrate salt would be useful for removing or ameliorating such symptoms as anxiety, agitation, excessive aggression, tension, and social or emotional withdrawal in psychotic patients. In addition, the tartrate salt may be useful in the blocking of serotonin-induced contractions of bronchial tissues and of blood vessels, arteries as well as veins. The tartrate salt of the present invention may also be useful as sedating-, anxiolytic-, anti-aggressive-, anti-stress-, muscular protectant-, and cardiovascular protectant agents and, consequently, it would be useful to protect warm-blooded animals, for example, in stress situations, e.g., during transport periods and the like situations.

Examples of movement disorders that can be treated according to the present invention include but are not limited to selected from Huntington's disease and dyskinesia associated with dopamine agonist therapy, Parkinson's disease, restless leg syndrome, and essential tremor.

Other disorders that can be treated according to the present invention are obsessive/compulsive disorders, Tourette's syndrome and other tic disorders.

This invention also provides a method for treating an anxiety disorder or condition in a mammal which method comprises administering to said mammal an amount of a tartrate salt of Formula I effective in treating said disorder or condition. Examples of anxiety disorders that can be treated according to the present invention include, but are not limited to, panic disorder; agoraphobia; a specific phobia; social phobia; obsessive-compulsive disorder, post-traumatic stress disorder; acute stress disorder; and generalized anxiety disorder.

This invention further provides a method of treating a drug addiction, for example an alcohol, amphetamine, cocaine, or opiate addiction, in a mammal, including a human, which method comprises administering to said mammal an amount of a tartrate salt of Formula I effective in treating drug addiction. A "drug addiction, as used herein, means an abnormal desire for a drug and is generally characterized by motivational disturbances such a compulsion to take the desired drug and episodes of intense drug craving.

This invention also provides a method of treating a disorder or condition comprising as a symptom a deficiency in attention and/or cognition in a mammal, including a human, which method comprises administering to said mammal an amount of a compound of formula I effective in treating said disorder or condition. The phrase "cognitive deficits" of "deficiency in attention and/or cognition" as used herein in refers to a subnormal functioning in one or more cognitive aspects such as memory, intellect, or learning and logic ability, in a particular individual relative to other individuals within the same general age population. "Cognitive deficits" or "deficiency in attention and/or cognition" also refers to a reduction in any particular individual's functioning in one or more cognitive aspects, for example as occurs in age-related cognitive decline.

Examples of disorders that comprise as a symptom a deficiency in attention and/or cognition that can be treated according to the present invention are dementia, for example Alzheimer's disease, multi-infarct dementia, alcoholic dementia or other drug-related dementia, dementia associated with intracranial tumors or cerebral trauma, dementia associated with Huntington's disease or Parkinson's disease, or AIDS-related dementia; delirium; amnestic disorder; post-traumatic stress disorder; mental retardation; a learning disorder, for example reading disorder, mathematics disorder, or a disorder of written expression; attention-deficit/hyperactivity disorder; age-related cognitive decline; cognitive deficits associated with psychoses, and cognitive deficits associated with schizophrenia.

This invention also provides a method of treating a mood disorder or mood episode in a mammal, including a human, comprising administering to said mammal an amount of a tartrate salt of Formula I effective in treating said disorder or episode. Examples.of mood disorders and mood episodes that can be treated according to the present invention include, but are not limited to, major depressive episode of the mild, moderate or severe type, a manic or mixed mood episode, a hypomanic mood episode; a depressive episode with atypical features; a depressive episode with melancholic features; a depressive episode with catatonic features; a mood episode with postpartum onset; post-stroke depression; major depressive disorder, dysthymic disorder; minor depressive disorder; premenstrual dysphoric disorder; post-psychotic depressive disorder of schizophrenia; a major depressive disorder superimposed on a psychotic disorder such as delusional disorder or schizophrenia; a bipolar disorder, for example bipolar I disorder, bipolar II disorder, and cyclothymic disorder.

This invention further provides a method of treating a neurodegenerative disorder or condition in a mammal, including a human, which method comprises administering to said mammal an amount of a tartrate salt of Formula I effective in treating said disorder or condition. As used herein, and unless otherwise indicated, a "neurodegenerative disorder or condition" refers to a disorder or condition that is caused by the dysfunction and/or death of neurons in the central nervous system. The treatment of these disorders and conditions can be facilitated by administration of an agent which prevents the dysfunction or death of neurons at risk in these disorders or conditions and/or enhances the function of damaged or healthy neurons in such a way as to compensate for the loss of function caused by the dysfunction or death of at-risk neurons. The term "neurotrophic agent" as used herein refers to a substance or agent that has some or all of these properties.

Examples of neurodegenerative disorders and conditions that can be treated according to the present invention include, but are not limited to, Parkinson's disease; Huntington's disease; dementia, for example Alzheimer's disease, multi-infarct dementia, AIDS-related dementia, and Fronto temperal Dementia; neurodegeneration associated with cerebral trauma; neurodegeneration associated with stroke, neurodegeneration associated with cerebral infarct; hypoglycemia-induced neurodegeneration; neurodegeneration associated with epileptic seizure; neurodegeneration associated with neurotoxin poisoning; and multi-system atrophy.

In one embodiment of the present invention, the neurodegenerative disorder or condition comprises neurodegeneration of striatal medium spiny neurons in a mammal, including a human. In a further embodiment of the present invention, the neurodegenerative disorder or condition is Huntington's disease.

In another embodiment of the present invention, the tartrate salt of the present invention may be used in the prophylaxis and/or treatment of sexual dysfunction. Sexual dysfunction (SD) is a significant clinical problem, which can affect both males and females. The causes of SD may be both organic as well as psychological. Organic aspects of SD are typically caused by underlying vascular diseases, such as those associated with hypertension or diabetes mellitus, by prescription medication and/or by psychiatric disease such as depression. Physiological factors include fear, performance anxiety and interpersonal conflict. SD impairs sexual performance, diminishes self-esteern and disrupts personal relationships thereby inducing personal distress. In the clinic, SD disorders have been divided into female sexual dysfunction (FSD) disorders and male sexual dysfunction (MSD) disorders (Melman *et al* 1999). FSD includes female sexual arousal disorder (FSAD), desire disorders such as hypoactive sexual disorder (lack of interest in sex), and orgasmic disorders such as anorgasmia (unable to achieve orgasm). Male sexual dysfunction (MSD) includes male erectile dysfunction (MED) and ejaculatory disorders such as an orgasmia (unable to achieve orgasm) or desire disorders such as hypoactive sexual desire disorder (lack of interest in sex).

The tartrate salt of the invention is beneficial for the prophylaxis and/or treatment of sexual dysfunction in the male (e.g. male erectile dysfunction - MED) and in the female - female sexual dysfunction (FSD), e.g. female sexual arousal disorder (FSAD).

In a further aspect, the present invention provides a method for treating lower urinary tract dysfunction by administering to a mammal a tartrate salt of Formula I in an amount effective to treat the disorder. Conditions of lower urinary tract dysfunction include overactive bladder, increased daytime frequency, nocturia, urgency, urinary incontinence (any condition in which there is an involuntary leakage of urine), including stress urinary incontinence, urge urinary incontinence and mixed urinary incontinence, overactive bladder with associated urinary incontinence, enuresis, nocturnal enuresis, continuous urinary incontinence, situational urinary incontinence such as incontinence during sexual intercourse, and lower urinary tract symptoms (LUTS) associated with benign prostatic hyperplasia (BPH).

The tartrate salt of Formula I of the present invention can be administered to a patient at dosage levels in the range of from about 0.1 mg to about 1,000 mg per day (preferably, about 1 mg to about 500 mg per day, more preferably, about 2.5 mg to about 250 mg per day, still more preferably about 5 mg to about 150 mg per day, and most preferably, about 60 mg to about 100 mg per day). For a normal adult human having a body weight of about 70 kg, a dosage in the range of from about 0.01 mg to about 2 mg per kilogram body weight is typically sufficient. However, some variability in the general dosage range may be required depending upon the age and weight of the subject being treated, the intended route of administration, the particular compound being administered and the like. The determination of dosage ranges and optimal dosages for a particular patient is well within the ability of one of ordinary skill in the art having the benefit of the instant disclosure. It is also noted that the tartrate salt of the present invention can be used in sustained release, controlled release, and delayed release formulations, which forms are also well known to one of ordinary skill in the art.

The tartrate salt of Formula I the invention may also be used in conjunction with other pharmaceutical agents for the treatment of the diseases/conditions described herein. Therefore, methods of treatment that include administering a tartrate salt of the present invention in combination with other pharmaceutical agents are also provided. Suitable pharmaceutical agents that may be used in combination with the compounds of the present invention include anti-obesity agents such as apolipoprotein-B secretion/microsomal triglyceride transfer protein (apo-B/MTP) inhibitors, 11β-hydroxy steroid dehydrogenase-1 (11β-HSD type 1) inhibitors, PYY₃₋₃₈ and analogs thereof, MCR-4 agonists, cholecystokinin-A (CCK-A) agonists, monoamine reuptake inhibitors (such as sibutramine), sympathomimetic agents, β₃ adrenergic receptor agonists, dopamine agonists (such as bromocriptine), melanocyte-stimulating hormone receptor analogs, cannabinoid 1 receptor antagonists (e.g., rimonabant), melanin concentrating hormone antagonists, leptins (the OB protein), leptin analogs, leptin receptor agonists, galanin antagonists, lipase inhibitors (such as tetrahydrolipstatin, i.e. orlistat), anorectic agents (such as a bombesin agonist), Neuropeptide-Y receptor antagonists (e.g., NPY Y5 receptor antagonists, such as the spiro compounds described in US Patent Nos. 6,566,367; 6,649,624; 6,638,942; 6,605,720; 6,495,559; 6,462,053; 6,388,077; 6,335,345; and 6,326,375; US Publication Nos. 2002/0151456 and 2003/036652; and PCT Publication Nos. WO 03/010175. WO 03/082190 and WO 02/048152), thyromimetic agents, dehydroepiandrosterone or an analog thereof, glucocorticoid receptor agonists or antagonists, orexin receptor antagonists, urocortin binding protein antagonists, glucagon-like peptide-1 receptor agonists, ciliary neurotrophic factors (such as Axokine™ available from Regeneron Pharmaceuticals, Inc., Tarrytown, NY and Procter & Gamble Company, Cincinnati, OH), human agouti-related proteins (AGRP), ghrelin receptor antagonists, histamine 3 receptor antagonists or inverse agonists, and neuromedin U receptor agonists. Other anti-obesity agents, including the preferred agents set forth hereinbelow, are well known, or will be readily apparent in light of the instant disclosure, to one of ordinary skill in the art.

Preferred are anti-obesity agents selected from the group consisting of orlistat, sibutramine, bromocriptine, ephedrine, leptin, rimonabant, pseudoephedrine, PYY₃₋₃₈ or an analog thereof, and 2-oxo-N-(5-phenylpyrazinyl)spiro-[isobenzofuran-1(3H).4'-piperidine]-carboxamide.

Other suitable pharmaceutical agents that may be administered in combination with the tartrate salt of the present invention include agents designed to treat tobacco abuse (e.g., nicotine receptor partial agonists, bupropion hypochloride (also known under the tradename Zyban™) and nicotine replacement therapies), ADD/ADHD treatment agents (e.g., Ritalin™, Strattera™, Concerta™ and Adderall™), and agents to treat alcoholism, such as opioid antagonists (e.g., naltrexone (also known under the tradename ReVia™) and nalmefene), disulfiram (also known under the tradename Antabuse™), and acamprosate (also known under the tradename Campral™)). In addition, agents for reducing alcohol withdrawal symptoms may also be co-administered, such as benzodiazepines, beta-blockers, clonidine, carbamazepine, pregabalin, and gabapentin (Neurontin™). Treatment for alcoholism is preferably administered in combination with behavioral therapy including such components as motivational enhancement therapy, cognitive behavioral therapy, and referral to self-help groups, including Alcohol Anonymous (AA). In addition to Zyban, other useful nicotine receptor partial agonists are described in US Patent Nos. 6,235,734; 6,410.550; and 6,462,035; all of which are incorporated herein by reference.

Other pharmaceutical gents that may be used in combination include antidepressants (e.g., fluoxetine hydrochloride (Prozac™)); and neuroprotective agents (e.g., memantine).

In another embodiment, a tartrate salt of the present invention is used in combination with cognitive improvement agents such as donepezil hydrochloride (Aricept™) and other acetylcholinesterase inhibitors; cannabinoid receptor 1 (CB1) antagonists; and alpha 7 nicotinic acetylcholine receptor agonists. Representative alpha 7 agonist compounds are listed in US Patent Nos. 6,911,543; 6,809,094; and 6,881,734, all of which are incorporated herein by reference.

According to a yet further aspect, the present invention additionally provides a method for the treatment and/or prevention of male sexual dysfunction via treatment with a combination of a tartrate salt of the present invention and at least one additional pharmaceutical agent. Preferred additional pharmaceutical agents used in treating male sexual dysfunction (e.g., male erectile dysfunction) include: (1) one or more dopaminergic agents (e.g. D2, D3 or D4 agonists and apomorphine); (2) one or more of an NPY (neuropeptide Y) (preferably an NPY-1 and/or NPY-5 inhibitor); (3) one or more of a melanocortin receptor agonist or modulator or melanocortin enhancer; (4) one or more of an NEP inhibitor; (5) one or more of a PDE inhibitor (preferably, a cGMP PDE-5 inhibitor); and (6) one or more of a bombesin receptor antagonist or modulator.

According to another aspect of the present invention, there is provided use of a tartrate salt of Formula I of the present invention and one or more additional active agents for the treatment of female sexual dysfunction (FSD). Preferably, the one or more additional active agents is/are selected from the group consisting of: estrogen receptor modulators (e.g., estrogen agonists and/or estrogen antagonists); testosterone replacement agents and/or testosterone (Tostrelle) and/or dihydrotestosterone and/or dehydroepiandrosterone (DHEA) and/or a testosterone implant; estrogen, estrogen and medroxyprogesterone or medroxyprogesterone acetate (MPA) (as a combination), or a combination of estrogen and a methyl testosterone hormone replacement therapy agent; one or more dopaminergic agents; one or more NPY (neuropeptide Y) inhibitors; one or more melanocortin receptor modulators or melanocortin enhancers; one or more NEP (neutral endopeptidase) inhibitors; one or more PDE (phosphodiesterase) inhibitors; and one or more bombesin receptor modulators.

In another aspect, the compounds of the invention can be used in combination with other agents for the treatment of lower urinary tract dysfunction. Such other agents include: muscarinic acetylcholine receptor antagonists such as tolterodine; alpha adrenergic receptor antagonists, in particular an alpha1 adrenergic receptor antagonist or an alpha2 adrenergic receptor antagonist; alpha adrenergic receptor agonists or partial agonists, in particular an alpha1 adrenergic receptor agonist or partial agonist, or an alpha2 adrenergic receptor agonist or partial agonist; serotonin and noradrenalin reuptake inhibitor (SNRI); noradrenalin reuptake inhibitor (NRI) such as reboxetine, either in its racemic or (S,S)-enantiomeric form; vanilloid receptor (VR) antagonists, such as capsaicin; alpha2delta ligand, such as gabapentin or pregabalin; beta3 adrenergic receptor agonists; 5HT1a receptor antagonists or 5HT1a receptor inverse agonists; prostanoid receptor antagonists, e.g. EP1 receptor antagonist.

The dosage of the additional pharmaceutical agent will be generally dependent upon a number of factors including the health of the subject being treated, the extent of treatment desired, the nature and kind of concurrent therapy, if any, and the frequency of treatment and the nature of the effect desired. The determination of dosage ranges and optimal dosages for a particular patient is also well within the ability of one of ordinary skill in the art having the benefit of the instant disclosure.

The present invention also relates to a method of treating a mammal suffering from schizophrenia or psychoses, comprising administering a tartrate salt of Formula I, in an amount that is effective in treating schizophrenia or psychoses, and an antipsychotic drug or pharmaceutically acceptable salt thereof. The tartrate salt of Formula I and the antipsychotic drug may be administered together or separately, simultaneously or at separate intervals. An embodiment of the present invention provides a pharmaceutical composition comprising a tartrate salt of Formula I, and an antipsychotic drug or pharmaceutically acceptable salt thereof.

The antipsychotic drug may be, for example, Chlorpromazine, Fluphenazine, Haloperidol, Loxapine, Mesoridazine, Molindone, Perphenazine, Pimozide, Thioridazine, Thiothixene, or Trifluoperazine. These drugs all have an affinity for the dopamine 2 receptor. The antipsychotic drug may also be, for example, Asenapine, Ziprasidone, Olanzapine, Clozapine, Risperidone, Sertindole, Quetiapine, Aripiprazole or Amisulpride.

The combinations may result in synergistic action allowing a lower dose of the atypical antipsychotic to be administered while achieving at least the same psychotropic effect as achieved with a standard dose of the atypical antipsychotic. The dosage of the atypical antipsychotic may be reduced by about 25-90%, for example, about 40-80% and typically about 50-70%. The reduction in amount of antipsychotic required will be dependent on the amount of the tartrate salt of Formula I given.

The selection of the dosage of each therapeutic agent is that which can provide relief to the patient as measured by a reduction or amelioration of symptoms associated with the disorder or condition of the patient. As is well known, the dosage of each component depends on several factors such as the potency of the selected specific compound, the mode of administration, the age and weight of the patient, the severity of the condition to be treated, and the like. Determining a dose is within the skill of the ordinary artisan. To the extent necessary for completeness, the synthesis of the components of the compositions and dosages are as described in the listed patents above or the Physicians' Desk Reference, 57th ed., Thompson, 2003 which are expressly incorporated herein by reference. Desirably, when ziprasidone is selected as the active agent, the daily dose contains from about 5 mg to about 460 mg. More preferably, each dose of the first component contains about 20 mg to about 320 mg of the ziprasidone, and even more preferably, each dose contains from about 20 mg to about 160 mg of ziprasidone. Pediatric dosages may be less such as for example in the range of about 0.5 mg to about 40 mg daily. This dosage form permits the full daily dosage to be administered in one or two oral doses, for example.

General outlines of the dosages for the atypical antipsychotics, and some preferred dosages, are provided herein. This list is not intended to be complete but is merely a guideline for any of the desired combinations of the present invention.

Olanzapine: from about 0.25 to about 100 mg, once/day; preferably, from about 1 to about 30 mg, once/day; and most preferably about 1 to about 25 mg once/day, Clozapine: from about 12.5 to about 900 mg daily; preferably, from about 150 to about 450 mg daily; Risperidone: from about 0.25 to about 16 mg daily; preferably, from about 2-8 mg daily; Sertindole: from about 0.0001 to about 1.0 mg/kg daily; Quetiapine: from about 1.0 to about 40 mg/kg given once daily or in divided doses; Asenapine: from about 0.005 to about 60 mg total per day, given as a single dose or in divided doses; Paliperidone: from about 0.01 mg/kg to about 4 mg/kg body weight, more preferably from about 0.04 to about 2 mg/kg body weight; Bifeprunox.

A preferred atypical antipsychotic used according to the invention is ziprasidone. Ziprasidone (5-[2-[4-(1,2-benzisothiazol-3-yl)piperazin-1-yl]ethyl]-6-chloroindolin-2-one) is a benzisothiazolyl piperazine atypical antipsychotic with in vitro activity as a 5-HT_{1A} receptor agonist and an inhibitor of serotonin and norepinephrine reuptake (U.S. Patent No. 4,831,031). The postsynaptic 5-HT_{1A} receptor has been implicated in both depressive and anxiety disorders (NM Barnes, T Sharp, 38 Neuropharmacology 1083-152,1999). Oral bioavailability of ziprasidone taken with food is approximately 60%, half-life is approximately 6-7 hours, and protein binding is extensive.

Ziprasidone is efficacious for the treatment of patients with schizophrenia and schizomood disorders, refractory schizophrenia, cognitive impairment in schizophrenia, affective and anxiety symptoms associated with schizoaffective disorder and bipolar disorder. The drug is considered a safe and efficacious atypical antipsychotic (Charles Caley & Chandra Cooper, 36 Ann. Pharmacother., 839-51; (2002).

The present invention is useful in treating mental disorders and conditions, the treatment of which is facilitated by the administration of ziprasidone. Thus, the present invention has application where ziprasidone use is indicated as, e.g., in U.S. Patent Nos. 6,245,766; 6,245,765; 6,387;904; 5,312,925; 4,831,031; and European EP 0901789 published March 17, 1999, all of which are incorporated herein by reference.

Other atypical antipsychotics which can be used include, but are not limited to:
Olanzapine, 2-methyl-4-(4-methyl-1-piperazinyl)-10H-thieno[2,3-b][1,5]-benzodiazepine. Olanizapine is a known compound and is described in U.S. Patent No. 5,229,382 as being useful for the treatment of schizophrenia, schizophreniform disorder, acute mania, mild anxiety states, and psychosis. U.S. Patent No. 5,229,382 is herein incorporated herein by reference in its entirety;
Clozapine, 8-chloro-11-(4-methyl-1-piperazinyl)-5H-dibenzo[b,e][1,4]diazepine.
   Clozapine is described in U.S. Patent No. 3,539,573, which is herein incorporated by reference in its entirety. Clinical efficacy in the treatment of schizophrenia is described (Hanes, et al., Psychopharmacol. Bull., 24, 62 (1988));
Risperidone, 3-[2-[4-(6-fluoro-1,2-benzisoxazol-3-yl)piperidino]ethyl]-2-methyl-6,7,8,9 -tetrahydro-4H-pyrido-[1,2-a]pyrimidin-4-one. Risperidone and its use in the treatment of psychotic diseases are described in U.S. Patent No. 4,804,663, which is herein incorporated by reference in its entirety;
Sertindole, 1-[2-[4-[5-chloro-1-(4-fluorophenyl)-1H-indol-3-yl]-1-piperidinyl]ethyl]-imidazolidin-2-one. Sertindole is described in U.S. Patent No. 4,710,500. Its use in the treatment of schizophrenia is described in U.S. Patent Nos. 5,112,838 and 5,238,945. U.S. Patent Nos. 4,710,500; 5,112,838; and 5,238,945 are herein incorporated by reference in their entireties;
Quetiapine, 5-[2-(4-dibenzo[b,f][1,4]thiazepin-11-yl-1-piperazinyl)ethoxy]ethanol. Quetiapine and its activity in assays which remonstrate utility in the treatment of schizophrenia are described in U.S. Pat. No. 4,879,288, which is herein incorporated by reference in its entirety. Quetiapine is typically administered as its (E)-2-butenedioate (2:1) salt.
Aripiprazole, 7-{4-[4-(2,3-dichlorophenyl)-1-piperazinyl]-butoxy}-3-,4-dihydro carbostyril or 1-{4-[4-(2,3-dichlorophenyl)-1-piperazinyl]-butoxy}-3,4-dihydro-2(1H)-quinolinone. Aripiprazole is an atypical antipsychotic agent used for the treatment of schizophrenia and described in U.S. Patent No. 4,734,416 and U.S. Patent No. 5,006,528, which are herein incorporated by reference in their entireties.
Amisulpride, which is described in U.S. Patent No. 4,401,822. U.S. Patent No. 4,401,822 is incorporated herein in its entirety.
Asenapine, trans-5-chloro-2-methyl-2,3,3a,12b-tetrahydro-1H-dibenz[2,3:6,7]-oxepino[4,5-c]pyrrole. Preparation and use of asenapine is described in U.S. Patent Nos. 4,145,434 and 5,763,476, the entire contents of which are incorporated herein by reference.
Patiperidone, 3-[2-[4-(6-fluoro-1,2-benzisoxazol-3-yl)-piperidinyl]ethyl]-6,7,8,9-tetrahydro-9-hydroxy-2-methyl-4H-pyrido[1,2-a]pyrimidin-4-one. Preparation and use of paliparidone is described, for example, in U.S. Patent Nos. 6,320,048; 5,158,952; and 5,254,556, the entire contents of which are incorporated herein by reference.
Bifeprunox, 2-[4-[4-(5-fluoro-1H-indol-3-yl)-3,6-dihydro-1(2H)-pyridinyl]butyl] -1H-isoindole-1,3(2H)-dione. Preparation and use of bifeprunox is described in U.S. Patent 6,225,312, which is incorporated in its entirety herein.

A preferred combination is ziprasidone with a tartrate salt of Formula I of the present invention.

## Claims

1. A tartrate salt of Formula 1: 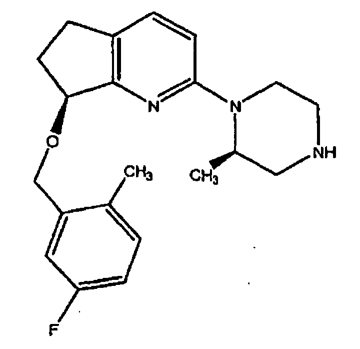 wherein the salt has characteristic X-ray powder diffraction peaks as measured with copper radiation of 2-Theta ± 0.2° of 3.5 °, 7.0 °, 15.6°, 18.1°, and 20.7 °.

2. The salt according to claim 1, wherein the salt has the characteristic X-ray powder diffraction pattern of FIG. 1.

3. A pharmaceutical composition comprising the salt according to claim 1 and a pharmaceutically acceptable carrier.

4. The tartrate salt of formula 1 as claimed in claim 1 for use as a medicament.

5. The tartrate salt of formula 1 as claimed in claim 1 for use in treating a 5-HT_{2c} receptor-mediated disease, condition, or disorder In a mammal.

6. The compound of Claim 5 wherein the disease, condition or disorder is selected from the group consisting of eating disorders, weight loss or control, obesity, diabetes insipidus, type II diabetes, depression, atypical depression, bipolar disorders, psychoses, schizophrenia, behavioral addictions, suppression of reward-related behaviors, substance abuse, addictive disorders, impulsivity, alcoholism, tobacco abuse, premenstrual syndrome or late luteal phase syndrome, migraine, panic disorder, anxiety, post-traumatic syndrome, dementia, seizure disorders, gastrointestinal disorders, attention deficit disorders or attention deficit hyperactivity disorders (ADD/ADHD), disruptive behavior disorders, impulse control disorders, borderline personality disorder, obsessive compulsive disorder, chronic fatigue syndrome, anorexia nervosa, disorders of sleep, autism, epilepsy, mutism, spinal cord injury, damage of the central nervous system, cardiovascular disorders, Parkinson's disease, Huntington's disease, dyskinesia associated with dopamine agonist therapy, restless leg syndrome, essential tremor, disorders that comprise as a symptom a deficiency in attention and/or cognition, a mood disorder or mood episode, a neurodegenerative disorder or condition, Tourette's syndrome, a tic disorder, male sexual dysfunction (MSD), female sexual dysfunction (FSD), and lower urinary tract dysfunction.

7. The compound of Claim 5 wherein the disease, condition, or disorder is selected from the group consisting of psychoses; schizophrenia; schizophreniform disorder; schizoaffective disorder; delusional disorder; substance-induced psychotic disorder; personality disorder of the paranoid type; and personality disorder of the schizoid type.

8. The compound of Claim 5 wherein the disease, condition or disorder is selected from the group consisting of anxiety; panic disorder; agoraphobia; a specific phobia; social phobia; obsessive-compulsive disorder; post-traumatic stress disorder; acute stress disorder; and generalized anxiety disorder.

9. The compound of Claim 5 wherein the disease, condition or disorder is selected from the group consisting of dementia; cognitive deficit symptoms of Alzheimer's disease; attention deficit symptoms of Alzheimer's disease; multi-infarct dementia, alcoholic dementia or other drug-related dementia, dementia associated with intracranial tumors or cerebral trauma, dementia associated with Huntington's disease or Parkinson's disease, or AlDS-related dementia; delirium; amnestic disorder; post-traumatic stress disorder; mental retardation; a learning disorder; attention-deficit/hyperactivity disorder; age-related cognitive decline; cognitive deficits associated with psychoses; and cognitive deficits associated with schizophrenia.

10. The compound of Claim 9 wherein the disease, condition or disorder is cognitive deficits associated with schizophrenia.

11. The compound of Claim 5 wherein the disease, condition or disorder is selected from the group consisting of a mood disorder, a mood episode, major depressive episode of the mild, moderate or severe type, a manic or mixed mood episode, a hypomanic mood episode; a depressive episode with atypical features; a depressive episode with melancholic features; a depressive episode with catatonic features; a mood episode with postpartum onset; post-stroke depression; major depressive disorder; dysthymic disorder; minor depressive disorder; premenstrual dysphoric disorder; post-psychotic depressive disorder of schizophrenia; a major depressive disorder superimposed on a psychotic disorder such as delusional disorder or schizophrenia; a bipolar disorder, e.g., bipolar I disorder, bipolar II disorder, and cyclothymic disorder.

12. The compound of Claim 5 wherein the disease, condition or disorder is selected from the group consisting of Parkinson's disease; Huntington's disease; neurodegeneration associated with Alzheimer's disease, multi-infarct dementia, AIDS-related dementia, and Fronto temperal Dementia; neurodegeneration associated with cerebral trauma; neurodegeneration associated with stroke, neurodegeneration associated with cerebral infarct; hypoglycemia-induced neurodegeneration; neurodegeneration associated with epileptic seizure; neurodegeneration associated with neurotoxin poisoning; and multi-system atrophy.

13. The compound of Claim 5 wherein the disease, condition or disorder is selected from the group consisting of eating disorders, weight loss or control, or obesity.

14. A pharmaceutical composition comprising the tartrate salt of formula 1 as claimed in Claim 1, an antipsychotic agent, and a pharmaceutically acceptable carrier.

15. The tartrate salt of formula 1 as claimed in claim 1 in combination with an antipsychotic agent for use in treating a 5-HT_{2c} receptor-mediated disease, condition, or disorder in a mammal.

## Patentansprüche

1. Tartratsalz der Formel I: 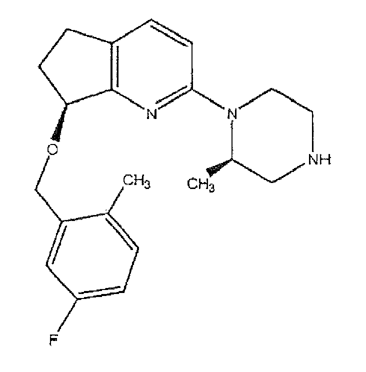 wobei das Salz charakteristische Pulver-Röntgenbeugungspeaks, wie sie mit Kupferstrahlung gemessen wurden, mit 2-Theta ± 0,2° von 3,5°, 7,0°, 15,6°, 18,1° und 20,7° hat.

2. Salz nach Anspruch 1, wobei das Salz das charakteristische Pulver-Röntgenbeugungsspektrum von Figur 1 hat.

3. Pharmazeutische Zusammensetzung, die das Salz nach Anspruch 1 und einen pharmazeutisch verträglichen Träger umfasst.

4. Tartratsalz der Formel I, wie es in Anspruch 1 beansprucht ist, zur Verwendung als Medikament.

5. Tartratsalz der Formel I, wie es in Anspruch 1 beansprucht ist, zur Verwendung bei der Behandlung einer (eines) 5-HT2_{c-}Rezeptor-vermittelten Erkrankung, Zustands oder Störung bei einem Säuger.

6. Verbindung nach Anspruch 5, wobei die Erkrankung, der Zustand oder die Störung ausgewählt ist aus der Gruppe, bestehend aus Essstörungen, Gewichtsverlust oder Kontrolle, Adipositas, Diabetes, Insipidus, Typ-II-Diabetes, Depression, atypischer Depression, bipolaren Störungen, Psychosen, Schizophrenie, Suchtverhalten, Supression von belohnungsbezogenem Verhalten, Substanzmissbrauch, Suchtstörungen, Impulsivität, Alkoholismus, Tabakmissbrauch, prämenstruellem Syndrom oder Syndrom der späten Lutealphase, Migräne, Panikstörung, Angst, posttraumatischem Syndrom, Demenz, Krampfleiden, gastrointestinalen Störungen, Aufmerksamkeitsstörungen oder Aufmerksamkeits- und Hyperaktivitätsstörungen (ADS/ADHS), expansiven Verhaltenstörungen, gestörte Triebkontrolle, Borderline-Persönlichkeitsstörung, obsessiv-kompulsive Störung, chronischem Müdigkeitssyndrom, Anorexia nervosa, Schlafstörungen, Autismus, Epilepsie, Mutismus, Rückenmarkverletzung, Schädigung des Zentralnervensystems, kardiovaskulären Störungen, Parkinson'scher Erkrankung, Huntington'scher Erkrankung, Dyskinesie, verbunden mit Dopamin-Agonisten-Therapie, Syndrom der unruhigen Beine, essentiellem Tremor, Störungen, die als Symptom Aufmerksamkeits- und/oder Wahrnehmungsdefizienz umfassen, Gemütsstörung oder Gemütsepisode, einer neurodegenerativen Störung oder eines neurodegenerativen Zustands, Tourette-Syndrom, nervösen Muskelzuckungen, männlicher sexueller Dysfunktion (MSD), weiblicher sexueller Dysfunktion (FSD) und Dysfunktion des unteren Harntrakts.

7. Verbindung nach Anspruch 5, wobei die Erkrankung, der Zustand oder die Störung ausgewählt ist aus der Gruppe, bestehend aus Psychosen; Schizophrenie; schizophreniformer Störung; schizoaffektiver Störung; wahnhafter Störung; Substanzinduzierter psychotischer Störung; Persönlichkeitsstörung des paranoiden Typs und Persönlichkeitsstörung des schizoiden Typs.

8. Verbindung nach Anspruch 5, wobei die Erkrankung, der Zustand oder die Störung ausgewählt ist aus der Gruppe, bestehend aus Angst; Panikstörung; Agoraphobie; einer spezifischen Phobie; sozialer Phobie; obsessiv-kompulsive Störung; posttraumatischer Stressstörung; akuter Stressstörung und generalisierter Angststörung.

9. Verbindung nach Anspruch 5, wobei die Erkrankung, der Zustand oder die Störung ausgewählt ist aus der Gruppe, bestehend aus Demenz; Wahrnehmungsdefizit-Symptomen der Alzheimer'schen Erkrankung; Aufmerksamkeitsdefizitsymptomen der Alzheimer'schen Erkrankung; Multiinfarkt-Demenz, Alkoholdemenz oder anderer Wirkstoff-bezogener Demenz, Demenz, assoziiert mit intrakranialen Tumoren oder Hirntrauma, Demenz, assoziiert mit Huntington'scher Erkrankung oder Parkinson'scher Erkrankung, oder AIDS-bedingter Demenz; Delirium; amnestischer Störung; posttraumatischer Stressstörung; mentaler Retardierung; einer Lernstörung; Aufmerksamkeitsdefizit/Hyperaktivitätsstörung; altersbedingter Wahrnehmungsverschlechterung; Wahrnehmungsdefiziten, assoziiert mit Psychosen, und Wahrnehmungsdefiziten, assoziiert mit Schizophrenie.

10. Verbindung nach Anspruch 9, wobei die Erkrankung, der Zustand oder die Störung kognitive Defizite, assoziiert mit Schizophrenie, ist.

11. Verbindung nach Anspruch 5, wobei die Erkrankung, der Zustand oder die Störung ausgewählt ist aus der Gruppe, bestehend aus einer Stimmungsstörung, einer Stimmungsepisode, einer typischen depressiven Episode des milden, moderaten oder schweren Typs, einer mänischer Episode oder einer Episode mit gemischter Stimmung, einer hypomanischen Stimmungsepisode; einer depressiven Episode mit atypischen Merkmalen; einer depressiven Episode mit melancholischen Merkmalen; einer depressiven Episode mit katatonischen Merkmalen; einer Stimmungsepisode mit Postpartum-Beginn; Depression nach Schlaganfall; typischer depressiver Störung; dysthymer Störung; kleinerer depressiver Störung; prämenstrueller dysphorischer Störung; post-psychotischer depressiver Störung von Schizophrenie; einer typischen depressiven Störung, die eine psychotische Störung, zum Beispiel wahnhafte Störung oder Schizophrenie, überlagert; einer bipolaren Störung, zum Beispiel Bipolar-I-Störung, Bipolar-II-Störung und zyklothyme Störung.

12. Verbindung nach Anspruch 5, wobei die Erkrankung, der Zustand oder die Störung ausgewählt ist aus der Gruppe, bestehend aus Parkinson'scher Erkrankung; Huntington'scher Erkrankung; Neurodegeneration, assoziiert mit Alzheimer'scher Erkrankung, Multiinfarkt-Demenz, AIDS-bedingter Demenz und frontotemporaler Demenz; Neurodegeneration, assoziiert mit zerebralem Trauma; Neurodegeneration, assoziiert mit Schlaganfall, Neurodegeneration, assoziiert mit Hirninfarkt; Hypoglykämie-induzierter Neurodegeneration, assoziiert mit epileptischem Krampf; Neurodegeneration, assoziiert mit Neurotoxinvergiftung, und Multisystematrophie.

13. Verbindung nach Anspruch 5, wobei die Erkrankung, der Zustand oder die Störung ausgewählt ist aus Gruppe, bestehend aus Essstörungen, Gewichtsverlust oder -kontrolle oder Adipositas.

14. Pharmazeutische Zusammensetzung, umfassend das Tartratsalz der Formel I, wie es in Anspruch 1 beansprucht ist, ein Antipsychotikum und einen pharmazeutisch verträglichen Träger.

15. Tartratsalz der Formel I, wie es in Anspruch 1 beansprucht ist, in Kombination mit einem Antipsychotikum zur Verwendung bei der Behandlung einer (eines) 5-HT_{2c}-Rezeptorvermittelten Erkrankung, Zustands oder Störung bei einem Säuger.

## Revendications

1. Sel tartrique de formule 1 : 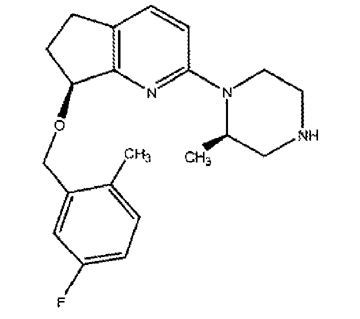 ce sel ayant des pics de diffraction des rayons X sur poudre caractéristiques, mesurés avec un rayonnement de cuivre, de 2-thêta + 0,22° à 3,5°, 7,0°, 15,6°, 18,1° et 20,7°.

2. Sel selon la revendication 1, ce sel ayant le diagramme de diffraction des rayons X sur poudre caractéristique de la FIG. 1.

3. Composition pharmaceutique comprenant le sel selon la revendication 1 et un support pharmaceutiquement acceptable.

4. Sel tartrique de formule 1 tel que revendiqué dans la revendication 1, destiné à une utilisation en tant que médicament.

5. Sel tartrique de formule 1 tel que revendiqué dans la revendication 1, destiné à une utilisation dans le traitement d'une maladie, d'un état ou d'un trouble médié(e) par le récepteur 5-HT_{2c} chez un mammifère.

6. Composé selon la revendication 5, dans lequel la maladie, l'état ou le trouble est sélectionné(e) dans le groupe consistant en les troubles alimentaires, la perte ou le contrôle de/du poids, l'obésité, le diabète insipide, le diabète de type II, la dépression, la dépression atypique, les troubles bipolaires, les psychoses, la schizophrénie, les addictions comportementales, la suppression des comportements associés à une récompense, la toxicomanie, les troubles addictifs, l'impulsivité, l'alcoolisme, le tabagisme, le syndrome prémenstruel ou le syndrome de la phase lutéale tardive, la migraine, le trouble panique, l'anxiété, le syndrome post-traumatique, la démence, les troubles épileptiques, les troubles gastro-intestinaux, les troubles du déficit de l'attention ou les troubles d'hyperactivité avec déficit de l'attention (ADD/ADHD), les troubles du comportement perturbateur, les troubles du contrôle des pulsions, le trouble de la personnalité borderline, le trouble obsessionnel compulsif, le syndrome de fatigue chronique, l'anorexie nerveuse, les troubles du sommeil, l'autisme, l'épilepsie, le mutisme, une lésion de la moelle épinière, une lésion du système nerveux central, les troubles cardio-vasculaires, la maladie de Parkinson, la maladie de Huntington, la dyskinésie associée à une thérapie par des agonistes de la dopamine, le syndrome des jambes sans repos, le tremblement essentiel, les troubles qui comprennent comme symptôme un déficit de l'attention et/ou cognitif, un trouble de l'humeur ou un épisode d'humeur, un trouble ou un état neurodégénératif, le syndrome de Tourette, un trouble de tic, la dysfonction sexuelle masculine (MSD), la dysfonction sexuelle féminine (FSD) et la dysfonction des voies urinaires inférieures.

7. Composé selon la revendication 5, dans lequel la maladie, l'état ou le trouble est sélectionné(e) dans le groupe consistant en les psychoses ; la schizophrénie ; le trouble schizophréniforme ; le trouble schizo-affectif ; le trouble délirant ; le trouble psychotique induit la toxicomanie ; le trouble de la personnalité de type paranoïaque ; et le trouble de la personnalité de type schizoïde.

8. Composé selon la revendication 5, dans lequel la maladie, l'état ou le trouble est sélectionné(e) dans le groupe consistant en l'anxiété ; le trouble panique ; l'agoraphobie ; une phobie spécifique ; la phobie sociale ; le trouble obsessionnel compulsif ; le trouble de stress post-traumatique ; le trouble de stress aigu ; et le trouble d'anxiété généralisée.

9. Composé selon la revendication 5, dans lequel la maladie, l'état ou le trouble est sélectionné(e) dans le groupe consistant en la démence ; les symptômes du déficit cognitif de la maladie d'Alzheimer ; les symptômes du déficit de l'attention de la maladie d'Alzheimer ; la démence multi-infarctus, la démence alcoolique ou la démence liée à d'autres substances, la démence associée à des tumeurs intracrâniennes ou à un traumatisme cérébral, la démence associée à la maladie de Huntington ou à la maladie de Parkinson, ou la démence liée au SIDA ; le délire ; le trouble amnésique ; le trouble de stress post-traumatique ; le retard mental ; un trouble de l'apprentissage le trouble de déficit de l'attention/hyperactivité ; le déclin cognitif lié à l'âge ; les déficits cognitifs associés aux psychoses ; et les déficits cognitifs associés à la schizophrénie.

10. Composé selon la revendication 9, dans lequel la maladie, l'état ou le trouble consiste en les déficits cognitifs associés à la schizophrénie.

11. Composé selon la revendication 5, dans lequel la maladie, l'état ou le trouble est sélectionné(e) dans le groupe consistant en un trouble de l'humeur, un épisode d'humeur, un épisode dépressif majeur de type léger, modéré ou sévère, un épisode d'humeur maniaque ou mixte, un épisode d'humeur hypomaniaque ; un épisode dépressif avec caractéristiques atypiques ; un épisode dépressif avec caractéristiques mélancoliques ; un épisode dépressif avec caractéristiques catatoniques ; un épisode d'humeur en début de post-partum ; la dépression post-accident vasculaire cérébral ; le trouble dépressif majeur ; le trouble dysthymique ; le trouble dépressif mineur ; le trouble dysphorique prémenstruel ; le trouble dépressif post-psychotique de la schizophrénie ; le trouble dépressif majeur superposé à un trouble psychotique tel qu'un trouble délirant ou la schizophrénie ; un trouble bipolaire, par exemple le trouble bipolaire I, le trouble bipolaire II et le trouble cyclothymique.

12. Composé selon la revendication 5, dans lequel la maladie, l'état ou le trouble est sélectionné(e) dans le groupe consistant en la maladie de Parkinson ; la maladie de Huntington ; la neurodégénérescence associée à la maladie d'Alzheimer, la démence multi-infarctus, la démence liée au SIDA et la démence fronto-temporale ; la neurodégénérescence associée à un traumatisme cérébral ; la neurodégénérescence associée à un accident vasculaire cérébral ; la neurodégénérescence associée à un infarctus cérébral ; la neurodégénérescence induite par une hypoglycémie ; la neurodégénérescence associée à une crise épileptique ; la neurodégénérescence associée à un empoisonnement neurotoxique ; et l'atrophie multisystématisée.

13. Composé selon la revendication 5, dans lequel la maladie, l'état ou le trouble est sélectionné(e) dans le groupe consistant en les troubles alimentaires, la perte ou le contrôle de/du poids, ou l'obésité.

14. Composition pharmaceutique comprenant le sel tartrique de formule 1 tel que revendiqué dans la revendication 1, un agent antipsychotique et un support pharmaceutiquement acceptable.

15. Sel tartrique de formule 1 tel que revendiqué dans la revendication 1 en combinaison avec un agent antipsychotique en vue d'une utilisation dans le traitement d'une maladie, d'un état ou d'un trouble médié(e) par le récepteur 5-HT_{2c} chez un mammifère.
